(19) Europäisches Patentamt
European Patent Office
Office européen des brevets

(11) **EP 2 551 087 A1**

(12) **EUROPÄISCHE PATENTANMELDUNG**

(43) Veröffentlichungstag:
30.01.2013  Patentblatt 2013/05

(21) Anmeldenummer: **11175837.1**

(22) Anmeldetag: **28.07.2011**

(51) Int Cl.:
*B29C 47/92* (2006.01)      *G01N 29/07* (2006.01)
*G01N 29/11* (2006.01)      *G01N 33/44* (2006.01)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(71) Anmelder: **Rhein Chemie Rheinau GmbH**
**68219 Mannheim (DE)**

(72) Erfinder:
• **Schröder, Andreas**
**69463 Weinheim (DE)**

• **Wawrzinski, Lars**
**68163 Mannheim (DE)**
• **Gräff, Ludwig**
**68519 Viernheim (DE)**

(74) Vertreter: **Siegers, Britta**
**Lanxess Deutschland GmbH**
**LIP Intellectual Property Rights**
**Gebäude Q 18**
**51369 Leverkusen (DE)**

(54) **Verfahren zur Bestimmung der Qualität von unvernetzten Kautschukmischungen sowie eine entsprechende Vorrichtung**

(57) Verfahren zur Bestimmung der Qualität einer unvernetzten Kautschukmischung (5) durch Messung der Transmission des Ultraschalls über die Prozesszeit, wobei die Messung bei Temperaturen > 25°C und Frequenzen zwischen 0,5 MHz und 20 MHz durchgeführt wird und Vorrichtung enthaltend eine Anordnung aus einem Extruder (1), mindestens einem Detektionsband aus mindestens 2 nebeneinander angeordneten Ultraschallsensorpaaren (4) und mindestens eine Auswerteeinheit (3) zur Durchführung des Verfahrens.

Fig.3

**Beschreibung**

Die Erfindung betrifft neue Verfahren zur Bestimmung der Qualität einer unvernetzten Kautschukmischung mittels Ultraschall und eine zu diesem Zweck geeignete Vorrichtung. Das erfindungsgemäße Verfahren ermöglicht sowohl die Detektion von partikulären Verunreinigungen als auch die Bestimmung der Güte der Verteilung von Feststoffen in der unvernetzten Kautschukmischung. Beide Kriterien sind qualitätsbestimmend für Kautschukmischungen.

Ultraschallanalytik für unvernetzte substanz/füllstoffhaltige Kautschukmischungen ist aufgrund der hohen Schalldämpfung nur sehr eingeschränkt möglich. Wünschenswert ist eine einfache und schnelle Methode zur Bestimmung von partikulären Verunreinigungen und deren Verteilungsgrad in einer unvernetzten Kautschukmischung.

Kautschuk wird für verschiedene technische Bauteile, wie Reifen, Motorlager, O-Ringe, etc. verwendet. Selbst einzelne grobe partikuläre Verunreinigungen > 100 $\mu$m, führen insbesondere unter dynamischer Belastung zu einem frühen Ausfall der Bauteile aus Gummi [A. Schröder et al., Kautschuk, Gummi Kunststoffe, 11, Seiten 584 bis 596, 2008]. Bei sehr kleinen dünnwandigen Bauteilen (1 mm) wie O-Ringen kann so eine partikuläre Verunreinigung zur Undichtigkeit führen. Bei Profilen müssen aufgrund der Optik der Bauteile partikuläre Verunreinigungen vermieden werden.

Der Verteilungsgrad der Substanzen im Kautschuk bestimmt dagegen auch das dynamischmechanische Wertebild der vernetzten Kautschukmischung und damit die Qualität der daraus bestehenden technischen Bauteile. Zum Beispiel erhöht sich die Hysterese und damit der Rollwiderstand eines Reifens, wenn die verstärkenden nanoskaligen Füllstoffe unzureichend deagglomeriert und verteilt werden [Payne, A.R.; Watson, W.F.: Rubber Chem. and Technol. 36 (1963) Nr. 1, S. 147 - 155].

Die Qualität einer unvernetzten Kautschukmischung wird bislang lediglich stichprobenartig (<0,1% der Gesamtmenge) überprüft. Es wird die Mooney-Viskosität des Kautschuks und Vernetzungskurven der Stichproben ermittelt. Nach Vulkanisation von Stichproben wird das Zug-Dehnungsverhalten der Mischung bestimmt [J. Schnetger Lexikon Kautschuktechnik, Heidelberg, 3. überarbeitete Auflage (2004), 388-391]. Einzelne grobe partikuläre Verunreinigungen in geringer Anzahl werden bei diesen Prüfungen nicht erkannt. Anhand der Messergebnisse kann zudem nicht unterschieden werden, ob es sich bei einer Abweichung von den Spezifikationen um partikuläre Verunreinigungen oder andere Fehler handelt. Zudem werden Stichproben lichtmikroskopisch untersucht [J. Schnetger Lexikon Kautschuktechnik, Heidelberg, 3. überarbeitete Auflage (2004), 448-449]. Hier können zwar einzelne Verunreinigungen erkannt werden, eine vollständige Qualitätskontrolle der Kautschukmischung wird aber nicht durchgeführt. Außerdem ist es günstig die Probe zu vulkanisieren. Die geringe Anzahl der Stichproben ist nicht repräsentativ. Außerdem ist die Beurteilung der Proben zur Deagglomeration von Rußen nach dem sogenannten Cabot-Verfahren subjektiv. Zudem werden die beschriebenen Qualitätskontrollen erst mehrere Minuten oder sogar Tage nach der Herstellung der Kautschukmischung manuell durchgeführt. Die Durchführung ist zeitaufwendig, da die Prüfung offline manuell durchgeführt wird.

Ultraschall wird zur Bestimmung von groben Fehlstellen in Materialien verwendet, die den Schall gut leiten, zum Beispiel bei Metallen oder Flüssigkeiten. Dabei wird in Reflektion nach dem Impuls-Echo Verfahren gemessen. Zudem kann mit Ultraschall die Partikelgrößenverteilung in niedrigviskosen Suspensionen, wie wässrige Suspensionen oder Polymerschmelzen, aus dem Frequenzspektrum ermittelt werden [US-A 5.121.629.]. In der Gummiindustrie wird Ultraschall ebenfalls zur Qualitätskontrolle von vernetzten Bauteilen verwendet [J. Schnetger Lexikon Kautschuktechnik, Heidelberg, 3. überarbeitete Auflage (2004), 557-558].

Unvulkanisierte Kautschukmischungen leiten den Ultraschall dagegen aufgrund ihrer viskoelastischen Eigenschaften und des häufig sehr hohen Anteiles von nanoskaligen Füllstoffen nur sehr schlecht [J. Schnetger Lexikon Kautschuktechnik, Heidelberg, 3. überarbeitete Auflage (2004), 557-558]. Das heißt der Ultraschall dringt nur in einer geringen Tiefe in eine unvernetzte Kautschukmischung ein. Es können daher dünnwandige unvulkanisierte Kautschukproben untersucht werden, durch welche der Schall gerade noch durchdringt. In J. Kirchhoff, et al., Gummi Kunststoffe, 55 (2002) 373-381 wird das Vernetzungsverhalten von Kautschukmischungen stichprobenartig an Proben mit 2 mm durchgeführt. In EP-2314442 A wird die Qualität einer Kautschukmischung hinsichtlich der Verteilung von Vernetzungschemikalien mittels eines Markers mit einer Partikelgröße < 100$\mu$m und einer Dichte > 2 g/cm$^3$ verwendet. Die Probendicke beträgt hier lediglich 4 mm.

Es ist daher im Stand der Technik kein Verfahren bekannt, mit dem sehr einfach, eindeutig, schnell und effektiv eine Qualitätskontrolle bei unvernetzten Kautschukmischungen möglich ist.

Aufgabe der vorliegenden Erfindung war daher ein neues und einfach zu handhabendes Verfahrens zur schnellen und verlässlichen Kontrolle der Qualität von unvernetzten Kautschukmischungen bereitzustellen sowie einer für diese Anwendung geeigneten Apparatur.

Die dieser Erfindung zugrunde liegenden Aufgabe konnte überraschenderweise dadurch gelöst werden, dass unvernetzte Kautschukmischungen während der Extrusion durch gleichzeitige Messung der Transmission des Ultraschalls bei höheren Temperaturen in einem geeigneten Frequenzbereich in Bezug auf Veränderungen, hervorgerufen durch partikuläre Verunreinigungen sowie Änderungen des Verteilungsgrades von Feststoffen darin, analysiert werden, ohne dass es der Anwesenheit von Markern o.ä. bedarf.

Gegenstand der vorliegenden Erfindung ist daher ein neues Verfahren zur Bestimmung der Qualität einer unvernetzten Kautschukmischung durch Messung der Transmission des Ultraschalls bei Temperaturen von mehr als 25°C, vorzugsweise mehr als 80° C, besonders bevorzugt bei mehr als 100°C und Frequenzen zwischen 0,5 MHz und 20 MHz. Das Ultraschallsignal wird dabei vorzugsweise über eine Strecke von ≥ 10 mm transmittiert.

Die Frequenz des Ultraschallsignals liegt dabei vorzugsweise zwischen 0,5 MHz bis 20 MHz, besonders bevorzugt zwischen 2 MHz und 10 MHz, ganz besonders bevorzugt zwischen 4 MHz und 7 MHz. Dabei wird ein großer Teil der Kautschukmischung von ≥ 1%, bevorzugt ≥ 10% besonders bevorzugt 100% der Kautschukmischung mit dem Ultraschall analysiert. Auf diese Weise ist eine repräsentative und nicht nur stichprobenartige Qualitätskontrolle von unvernetzten Kautschukmischungen möglich.

Auf diese Art und Weise gelingt es, aussagekräftige Signale über partikuläre Verunreinigungen sowie Änderungen des Verteilungsgrades von Feststoffen und der Deagglomeration in einer Kautschukmischung zu detektieren und so die Qualität der unvernetzten Kautschukmischung zu kontrollieren.

Als Kautschuke eignen sich insbesondere Naturkautschuk (NR), Isoprenkautschuk (IR), StyrolButadien-Kautschuk (SBR), Butadienkautschuk (BR), Isopren-isobutylen-Kautschuk (IIR), Polychloroprenkautschuk (CR), Acrylnitril-Butadien-Kautschuk (NBR), hydrierter Acrylnitril-Butadien-Kautschuk (HNBR), carboxylierter Acrylnitril-Butadien-Kautschuk (XNBR), hydrierter carboxyliertere Acrylnitril-Butadien-Kautschuk (HXNBR), Ethylen-Propylen-DienKautschuk (EPDM), Ethylen-Propylen-Kautschuk (EPM), Fluorkautschuk (FKM), Perfluorierter Fluorkautschuk (FFKM), Acrylat-Ethylen-Kautschuk (AEM), Acrylatkautschuk (ACM), Ethylen-Methylen-Acrylatkautschuk (EMA), chloriertes Polyethylen, chlorsulfoniertes Polyethylen, Ethylen-Vinylacetat-Kautschuk (EVA), Siliconkautschuk, Fluro-SiliconKautschuk, Ethylen-Epichlorhydrin Kautschuk (ECO), Epichlorhydrin-Kautschuk (CO) und/oder Polyurethan-Kautschuk (PU).

Bei der Kautschukmischung handelt es sich vorzugsweise um Mischungen von Kautschuken mit Füllstoffen, Vernetzungsmitteln, Alterungsschutzmitteln, Weichmacher und/oder weiteren Hilfsmitteln.

Füllstoffe im Sinne der Erfindung sind z.B. helle anorganische Füllstoffe, wie z.B. Glimmer, Kaolin, Kieselerde, Kieselsäure, Kreide, Talkum, Kohlenstoff-Füllstoffe, wie z.B. Ruß, Graphit, Carbon Nanotubes, magnetisierbare Füllstoffe wie Carbonyl-Eisenpulver, Eisenoxide, Ferrite und/oder Fasern wie z.B. Aramidfaserpulpe, Kohlefasern.

Vernetzungsmittel im Sinne der Erfindung sind:

Netzknotenbildner, wie z.B.

- Schwefel (löslich oder unlöslich) und/oder Schwefelspender, wie z.B. Dithiomorpholine (DTDM), Tetramethylthiuramdisulphide (TMTD), Tetraethylthiuramdisulphid (TETD), Dipentamethylenthiuramterasulphide (DPTT), Phosphorylpolysulfide, wie z.B. Rhenocure® SDT/S der Firma Rhein Chemie Rheinau GmbH und/oder
- Peroxide, wie z.B. Di-tert.Butyl-Peroxide, Di-(tert,Butyl-Peroxy-Timethyl-Cyclohexane, Di-(tert.-Butyl-Peroxy-Isopropyl)benzene, Dicumylperoxide, Dimethyl-Di(tert.Butyl-Peroxy) Hexine, Butyl-Di-(tert,Butyl-Peroxy-)Valerate,
- Resorcinol, Aldehyd-Amin-Kondensationsprodukte, wie z.B. Hexamethylentetramine, Resorcinol-Formaldehyd-Vorkondensate und/oder Vulkanisationsharze, wie zum Beispiel Halomethylphenolharz,
- Chinondioxime
- Bisphenole,

Beschleuniger, wie z.B.

- Carbamate bzw. Triazine, wie z.B. Hexamethylen-diamin-carbamat (HMDC), organische Triazine,
- Thiazole, wie z.B. 2-Mercapto-benzothiazol (MBT), Zink-mercaptobenzothiazol (ZnMBT), Thiadiazole (TDD),
- Sulfenamide, wie Cyclohexyl-benzothiazol-Sulphenamide (CBS), Di-benzothiazyl-disulphid (MBTS), Butyl-benzothiazole-Sulphenamide (TBBS), Dicyclohexyl-Benzothiazol-Sulphenamid (DCBS), 2-(4-Morpholinylmercapto)-benzothiazol (MBS),
- Thiurame, wie Tetramethyl-thiuram-monosulphid (TMTM), Tetraethyl-thiuram-disulphid (TETD), Tetramethyl-thiuram-disulphid (TMTD), Tetrabenzylthiuram Disulphid (TBTD), Dipentamethylene-Thiuram-Tetra(Hexa)-Sulphid (DPTT),
- Dithiocarbamate, wie Zn-Dimethyldithiocarbamate (ZDMC), Cu-Dimethyldithiocarbamate, Bi-Dimethyldithio-carbamate, Zn-Diethyldithiocarbamate (ZDEC), Tellurdiethyldithiocarbamate (TDEC), Zn-Dibuthyldithiocarbamate (ZDBC), Zn-Ethyl-Phenyl-Dithiocarbamate (ZEPC), Zn-Dibenzyl-Dithiocarbamate (ZBEC), Ni-Dibuthyl-Dithiocarbamate (NBC), Selendiethyldithiocarbamate (SeEDC), Selendimethyldithiocarbamate (SeDMC), Tellurdiethyldithiocarbamate (TeEDC),
- Thiophosphat- und Dithiophosphat, wie z.B. Zink-O,O-di-n-butyl-dithiophosphat (ZBDP), Zink-O-butyl-O-hexyl-dithiophosphat, Zink-O, O-diisooctyl-dithiophosphat (ZOPD), Dodecylammonium-diisooctyl-dithiophosphat (AOPD), wie z.B. die Rhenogran®-Typen ZDT, ZAT, ZBOP der Firma Rhein Chemie Rheinau GmbH,

- Harnstoff/Thioharnstoffe, wie z.B. Ethylenthioharnstoff (ETU), N,N,N'N'-Tetramethylthioharnstoff (TMTU), Diethylthioharnstoff (DETU), Dibutylthioharnstoff (DBTU), 3-(3,4-Dichlorphenyl)-1,1-dimethylharnstoff (Diuron) etc., und/oder
- Xanthogenatbeschleuniger, wie z.B. Zinkisopropylxanthogenat (ZIX),
- Guanidine, wie z.B. Diphenylguanidin (DPG) und/oder N',N-di-ortho-tolyl-Guanidin (DOTG) und die Guanidine-frei Ersatzbeschleuniger, wie Rhenogran ® XLA 60,

Verzögerer, wie z.B.

- N-Nitrososdiphenylamin, N-Cylcohexylthiophthalimid (CPT), wie z.B. Vulkalent® G), Sulfonamidderivate (wie z.B. Vulkalent ® E/C), Phthalsäureanhydrid (Vulkalent® B/C), wobei die Vulkalent®- Typen beider der Firma Lanxess Deutschland GmbH erhältlich sind, sowie Benzoesäureanhydrid.

Alterungsschutzmittel im Sinne der Erfindung sind z.B. verfärbende und nicht verfärbende Alterungsschutzmittel, wie z.B. Paraphenylendiamine, Isopropylphenylparaphenylendiamin (IPPD), Para-Phenylen-Diamin (6PPD), N,N-ditoly-p-phenylendiamine (DTPD), etc., Amine, wie z.B. Trimethyl-1,2-dihydrochinolin (TMQ), (Phenyl)amine]-1,4-naphthalendion (PAN), Bis (4-octylphenyl)amin (ODPA), styrolisiertes Diphenylamin (SDPA), Mono- und Bisphenole, wie z.B. 2,2'-Methylen-bis-(4-methyl-6-tert.butylphenol(BPH), 2,2'-Isobutyliden-bis-(4,6-dimethyl-phenol) (NKF), 2,2'-Dicyclopentadienyl-bis-(4-methyl-6-tert.-butyl-phenol) (SKF), 2,2'-Methylen-bis-(4-methyl-6-cyclohexyl-phenol (ZKF), 2,6-Di-tert.-butyl-p-kresol (BHT), substituiertes Phenol (DS), styrolisierte Phenole (SPH), Mercatpbenzimidazole, wie z.B. 2-Mercaptobenzimidazol (MBI), 2-Mercaptomethyl-benzimidazole (MMBI), Zink-4- und -5-methyl-2-mercapto-benzimidazole (ZMMBI), etc., Olefine, paraffinische und/oder aromatische Weichmacher. Die Zusammensetzung wird dabei auf das gewünschte Endprodukt abgestimmt.

Weichmacher im Sinne der Erfindung sind z.B. langkettige Ester und/oder Ether, wie Thioester, Phthalsäureester, Alkylsulfonsäureester, Adipinsäureester, Sebacinsäuresester, Dibenzylether, und/oder Mineralöle (paraffinische, aromatische naphthenische oder synthetische Öle).

Hilfsmittel im Sinne der Erfindung sind z.B. Dispergierhilfsmittel, wie z.B. Fettsäuren, Stearinsäure und/oder Ölsäure und/oder Aktivatoren, wie zum Beispiel Zinkoxid, Bleioxid, Bismuthoxid, Lithiumcarbonat, Natriumcarbonat und/oder Calciumhydroxid, Magnesiumoxid, Flammschutzmittel wie Antimonoxid, Kopplungsmittel wie Silane, etc..

Bei allen vorgenannten Produkten handelt es sich um handelsübliche Produkte, die gegebenenfalls auch in granulierter Form zum Beispiel als polymergebundene Additive und/oder als die in EP2314442A beschriebenen Vernetzerbatche eingesetzt werden.

Bei den unvernetzten Kautschukmischungen handelt es sich um Masterbatches, Grundmischungen und vernetzbare Kautschukmischungen.

Masterbatches enthalten vorzugsweise:

2,5 bis 90 Gew.-% Kautschuk
0 bis 50 Gew.-% Weichmacher
0 bis 97,5 Gew.-% Füllstoffe
0 bis 80 Gew.-% Vernetzungsmittel
0 bis 20 Gew.-% Dispergierhilfsmittel
0 bis 80% Gew.-% Aktivatoren und
0 bis 50 Gew.-% Alterungsschutzmittel.

Grundmischungen enthalten vorzugsweise

100 phr Kautschuk
0 bis 100 phr Weichmacher
0 bis 500 phr Füllstoffe
0 bis 30 phr Hilfsmittel und
0 bis 10 phr Alterungsschutzmittel.

Vernetzbare Kautschukmischungen sind Grundmischungen die zusätzlich 0 bis 20 phr Vernetzungsmittel enthalten.

Bevorzugt sind vernetzbare Kautschukmischungen.

Besonders bevorzugt sind vernetzbare Kautschukmischungen, die unter anderen 30 bis 90 phr Ruß und/oder Kieselsäure, 3 bis 7 phr Zinkoxid sowie 0,5 phr bis 4 phr Schwefel und 1 bis 5 phr Beschleuniger enthalten.

Die Herstellung der Masterbatches, der Grundmischung und der vernetzbaren Kautschukmischung erfolgt vorzugsweise nach den, dem Fachmann geläufigen Verfahren, wie diese beispielsweise beschrieben sind in PCT/EP2009/058041. Füllstoffe, Hilfsmittel, Vernetzungsmittel und/oder Alterungsschutzmittel werden dabei zusammen mit dem Kautschuk in einem Mischaggregat vermischt. Geeignete Mischaggregate sind zum Beispiel Innenmischer, Walzwerke, Extruder.

In einer weiteren Ausführungsform der Erfindung wird in einem ersten Schritt die Grundmischung in einem Mischaggregat hergestellt. Dabei können Temperaturen von mehr als 130°C erreicht werden. In einem zweiten Herstellungsschritt werden nach Abkühlen der Grundmischung auf Temperaturen kleiner 130°C Vernetzungsmittel zur Grundmischung in einem weiteren Mischaggregat hinzugegeben. Ein Teil der Vernetzungsmittel kann dabei schon im ersten Herstellungsschritt zur Grundmischung hinzugegeben werden.

Besonders bevorzugt sind dabei kontinuierliche Verfahren, bei denen die Masterbatche, Grundmischungen oder auch die vernetzbare Kautschukmischungen in einem Extruder hergestellt werden.

Ganz besonders bevorzugt ist das in DE-A-102008040138 beschriebene Herstellungsverfahren für vernetzbare Kautschukmischungen, in dem die Grundmischung in einem diskontinuierlichen Kneterprozess hergestellt wird, und Vernetzungsmittel in Form der in beschriebenen EP-A-2314442 beschriebenen Vernetzerbatche zur Grundmischung hinzugefügt werden und die Vernetzerbatche in einem kontinuierlichen Prozess mit einem Extruder mit der Grundmischung vermischt werden.

Die Verunreinigungen sind in den verwendeten Rohstoffen, wie Kautschuk, Füllstoffe, Alterungsschutzmittel, Vernetzungsmittel und/oder Hilfsmittel, enthalten und/oder entstehen während des Mischprozesses und/oder werden unbeabsichtigt hinzugefügt.

Verunreinigungen, die in den verwendeten Rohstoffen enthalten sind, sind zum Beispiel unzureichend dispergierte Agglomerate von Füllstoffen, Stippen aus Vernetzungsmittel, Hilfsmittel und Alterungsschutzmitteln bevorzugt mit hohem Schmelzpunkt von mehr als 100°C, Grit zum Beispiel aus der Produktion von Rußen, Verunreinigungen im Kautschuk besonders im Naturkautschuk wie zum Beispiel kleine Steinchen, Holzsplitter etc..

Verunreinigungen, die während des Mischprozesses der unvernetzten Kautschukmischung entstehen sind zum Beispiel anvernetzte Rückstände von Kautschukmischungen in Mischaggregaten, Metallabrieb aus den Mischaggregaten.

Verunreinigungen, die unbeabsichtigt hinzugefügt werden, sind zum Beispiel Holzsplitter von Paletten, Kunststoffspäne, kleine Schrauben etc..

Die partikulären Verunreinigungen weisen dabei bevorzugt einen Durchmesser von > 10 $\mu$m, bevorzugt > 100 $\mu$m und ganz besonders bevorzugt > 500 $\mu$m auf. Die partikulären Verunreinigungen sind dabei Feststoffe mit einem Schmelzpunkt > 80°C, bevorzugt > 100°C, besonders bevorzugt > 120°C, ganz besonders bevorzugt > 150°C.

Unterschiede in dem Verteilungsgrad von Füllstoffen in der unvernetzten Kautschukmischung resultieren auf schlechter Vermischung der Feststoffe und/oder deren Deagglomeration.

In einer Ausführungsform des erfindungsgemäßen Verfahrens werden die partikulären Verunreinigungen sowie Unterschiede im Verteilungsgrad in unvernetzten Kautschukmischungen mit mindestens einem Paar, vorzugsweise mit mindestens einem Detektionsband von wenigstens zwei Paaren Ultraschallsensoren detektiert. Die jeweiligen Sensoren eines Paares sind gegenüberliegend, die Paare sind in Reihe nebeneinander angeordnet. Der Abstand zwischen den Sensoren eines Paares beträgt vorzugsweise $\geq$ 10 mm. Ein Ultraschallsensor eines Paares fungiert dabei als Sender und der andere Sensor als Empfänger des Ultraschallsignals. Im Spalt zwischen den Sensoren befindet sich die unvernetzte Kautschukmischung. Dabei besteht vorzugsweise ein direkter Kontakt zwischen Sensoren und unvernetzter Kautschukmischung. Der Spalt ist an den Seiten begrenzt, wodurch ein Fließkanal vorzugsweise mit einer Höhe von $\geq$ 10 mm, besonders bevorzugt mit einer Höhe von > 20 mm ganz besonders bevorzugt mit einer Höhe von 30 mm entsteht. Vorzugsweise beträgt die Breite des Fließkanals $\geq$ 20 mm, besonders bevorzugt $\geq$ 50 mm, ganz besonders bevorzugt $\geq$ 100 mm. Die unvernetzte Kautschukmischung durchläuft mit einer Geschwindigkeit von vorzugsweise 0,1 m/min., besonders bevorzugt 1 m/min., ganz besonders bevorzugt 10 m/min., das Detektionsband. Hierfür wird vorzugsweise ein Extruder verwendet. Gleichzeitig wird die Intensität des transmittierten Ultraschallsignals am Empfänger über die Prozesszeit gemessen. Mit Hilfe einer Ultraschallprüfelektronik und einer Auswerteeinheit, wie zum Beispiel ein Oszilloskop oder bevorzugt ein Rechner, wird das Ultraschallsignal aufgezeichnet und ausgewertet. Beim Passieren einer grobpartikulären Verunreinigung reduziert sich die Intensität des Ultraschallsignals am Empfänger durch Streuung bzw. Reflektion der Schallwellen an dieser grobpartikulären Verunreinigung.

In einer bevorzugten Ausführungsform des erfindungsgemäßen Verfahrens wird das Ultraschallsignal in Form von Nadelimpulsen oder Paketen von Nadelimpulsen, sogenannten Bursts, ausgesandt. Der zeitliche Abstand zwischen den einzelnen Nadelimpulsen bzw. Bursts beträgt vorzugsweise mindestens $\leq$ 1 s, besonders bevorzugt mindestens $\leq$ 100 ms. Zusätzlich zu der Transmission der Intensität wird die Laufzeit, welche das Signal benötigt, um vom Sender zum Empfänger zu gelangen, gemessen. Aus der bekannten Wegstrecke wird die Schallgeschwindigkeit ermittelt. Beim Passieren von grobpartikulären Verunreinigungen wird die Schallgeschwindigkeit nur wenig verändert. Beim Passieren von kleineren Partikeln, zum Beispiel gut deagglomerierte Füllstoffeinheiten < 1 $\mu$m, wird zudem die Schallgeschwindigkeit verändert. So kann zwischen grob partikulären und anderen Verunreinigungen unterschieden werden.

Einzelne Verunreinigungen in der unvernetzten Kautschuk führen zu einer verminderten Signalintensität (Peak) am Empfänger. In einer weiteren bevorzugten Ausführungsform des erfindungsgemäßen Verfahrens wird aus der Dauer der Reduktion der Signalintensität und der Fließgeschwindigkeit die Größe der grobpartikulären Verunreinigungen ermittelt. Aus der Zahl der Peaks und deren Breite kann eine Partikelgrößenverteilung mit der Auswerteeinheit ermittelt werden. Fehlerhafte Bereiche der Kautschukmischung können nach Austritt aus dem Fließkanal entfernt werden.

In einer weiteren bevorzugten Ausführungsform des erfindungsgemäßen Verfahrens wird der Extruder gleichzeitig als Mischaggregat für die Kautschukmischung verwendet.

In einer bevorzugten Ausführungsform des erfindungsgemäßen Verfahrens durchläuft der Kautschuk ein Detektionsband aus mindestens einem Ultraschallsensorpaar, die über die gesamte Breite des Fließkanals angeordnet sind.

In einer ganz besonders bevorzugten Ausführungsform der Erfindung durchläuft die Kautschukmischung zwei Detektionsbänder, die leicht versetzt angeordnet sind. Hierdurch wird gewährleistet, dass die komplette Kautschukmischung analysiert wird.

Gegenstand der Erfindung ist auch eine Vorrichtung zur Durchführung des erfindungsgemäßen Verfahrens, enthaltend eine Anordnung aus einem Extruder (1), mindestens einem Detektionsband aus mindestens einem Ultraschallsensorpaar (4) und dem Fließkanal (5) sowie einer Auswerteeinheit (3).

Die erfindungsgemäße Vorrichtung wird vorzugsweise in den nachstehenden Figuren beschrieben, ohne dabei auf diese beschränkt zu sein.

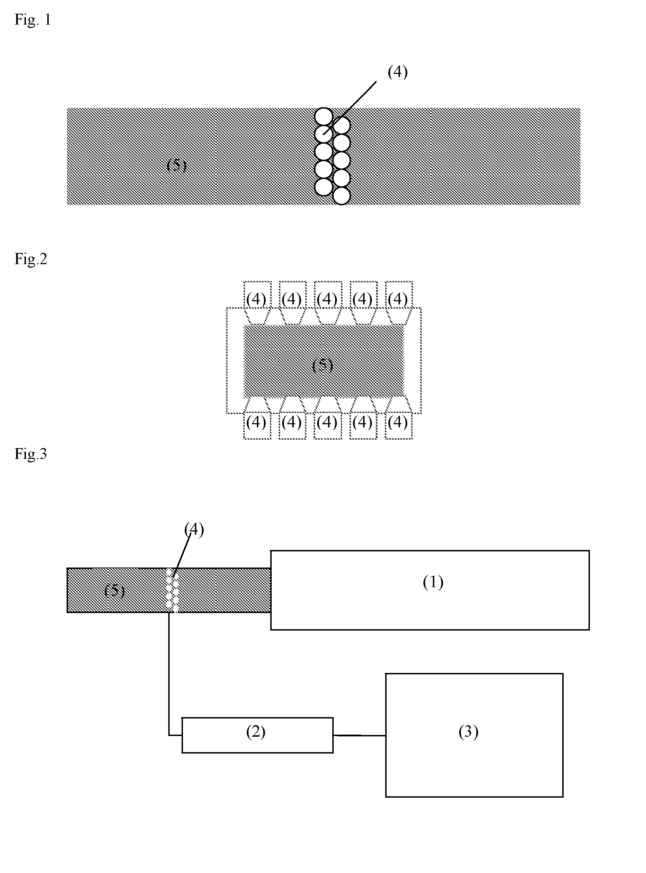

Fig. 1 beschreibt exemplarisch das Detektionsband, bestehend aus den Ultraschallsensorpaaren (4), d.h. pro Paar jeweils ein Sender und ein Empfänger und dem Fließkanal (5) in Aufsicht, in Fig. 2 ist die Anordnung in Fig. 1 in Seitenansicht dargestellt. Fig. 3 zeigt eine Anordnung aus einem Extruder (1), dem Detektionsband, bestehend aus den Ultraschallsensorpaaren (4) und dem Fließkanal (5) und der Ultraschallprüfelektronik (2) sowie einem Rechner als Auswerteeinheit (3).

Gegenstand der Erfindung ist zudem die Verwendung der erfindungsgemäßen Vorrichtung zur Bestimmung der Qualität in einer unvernetzten Kautschukmischung und zur Herstellung von unvernetzten Kautschukmischungen.

Ebenfalls mitumfasst von der Erfindung ist die Verwendung der nach dem erfindungsgemäßen Verfahren hergestellten unvernetzten Kautschukmischung für Reifen, Profile, O-Ringe und Bauteile zur Vibrationskontrolle.

Die nachfolgenden Beispiele und Figuren dienen der Erläuterung der Erfindung, ohne dabei limitierend zu wirken.

**Ausführunssbeispiele**

**Beispiel 1:**

Eine Grundmischung, bestehend aus 100 phr Naturkautschuk SMR10, 50 phr Ruß N550, 5 phr Weichmacher Naftolen ZD, 5 phr ZnO, 1 phr Stearinsäure, wurde mit einem Innenmischer hergestellt. Ca. 1 kg dieser Mischung wurde mit einem Einwellenextruder der Firma Rubicon EEK32.12L durch einen Fließkanal extrudiert. Der Fließkanal hat eine Länge von 300 mm, eine Höhe von 20 mm und eine Breite von 15 mm. In der Mitte des Fließkanals befindet sich ein Detektionsband, bestehend aus einem Paar Ultraschallsensoren K4V1 der Firma GE Sensing & Inspection Technologies GmbH mit einer mittleren Frequenz von 4 MHz. Der Schwinger des Ultraschallsensors besitzt dabei einen Durchmesser von 7 mm. Es wurde alle 100 ms ein Nadelimpuls von dem einen Sensor ausgesandt und von dem anderen Sensor empfangen. Die Sensoren wurden über die Prüfelektronik USLT 2000 der Firma GE Sensing & Inspection Technologies GmbH gesteuert und die empfangenen Messsignale über die Prüfelektronik zu einem Rechner gesandt, wo diese verarbeitet wurden. Die Temperatur des Extruders sowie des Fließkanals wurde auf 100°C temperiert. Die Drehzahl des Extruders betrug 20 U/min. Der Durchsatz betrug ca. 100 g/min. Hieraus ergab sich eine Extrusionsgeschwindigkeit von ca. 0,25 m/min. Die mittlere Verweilzeit vom Einzug bis zum Detektionsband betrug ca. 2 min.

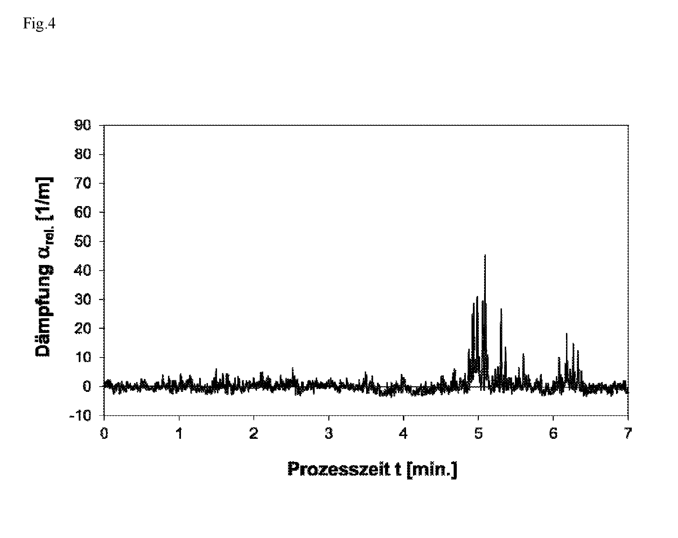

Die Messdaten sind aus den Figur 4 ersichtlich.

In Fig. 4 ist der relative Dämpfungskoeffizient $\alpha_{rel.}$ gegen die Prozesszeit t der Mischung aufgetragen. Der rel. Dämpfungskoeffizient $\alpha_{rel.}$ ist der normierte negative natürliche Logarithmus des Quotienten aus der transmittierten Intensität I(t) des Ultraschallsignals zum Zeitpunkt t und der mittleren transmittierten Intensität im Zeitbereich t = 0 bis t = 5 min., das heißt der mittleren transmittierten Intensität $I_{mittel}$ der Kautschukgrundmischung ohne Verunreinigungen. Die Wegstrecke s des Ultraschall entspricht der Probendicke.

$$\alpha_{rel.}\,(t) = -\ln(I(t)/I_{mittel})/s$$

Zum Zeitpunkt t = 3 min. werden ca. 0,5 g Kunststoffspäne mit einer Partikelgröße von ca. 2 mm bis 5 mm unregelmäßig für ca. 2 min. hinzugegeben. Nach ca. 2 min. Verweilzeit im Extruder wurden starke Schwankungen des Dämpfungskoeffizienten beobachtet (Fig. 4). Die Dämpfung nahm aufgrund der Streuung des Ultraschallsignals an den grobpartikuläre Kunststoffspänen stark zu. Verunreinigungen, wie in diesem Fall die Kunststoffspäne, sind mit dem erfindungsgemäßen Verfahren eindeutig detektierbar.

**Beispiel 2:**

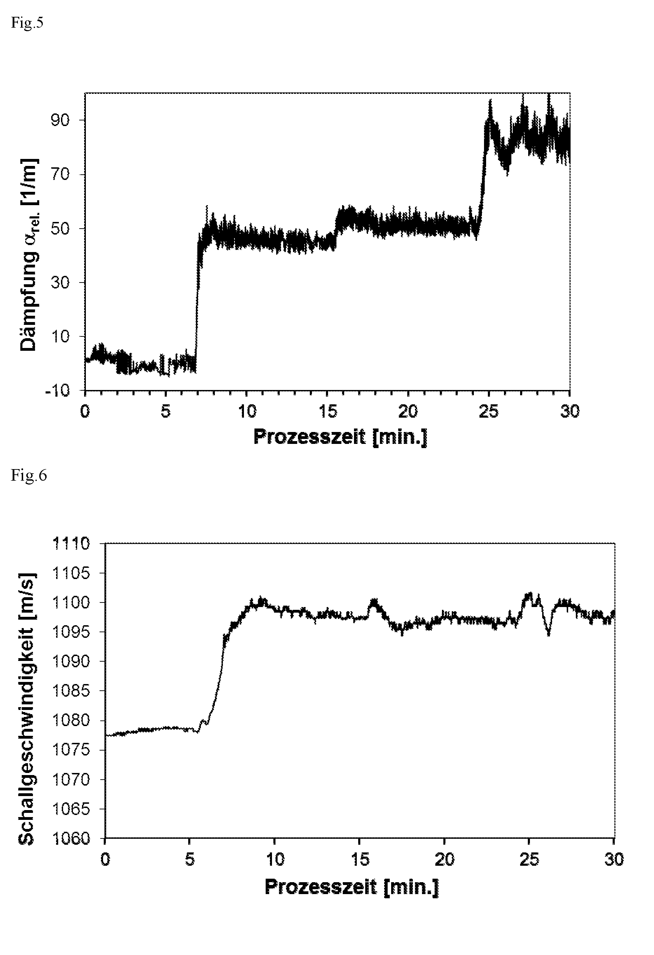

Es wurden vier Kautschukgrundmischungen mit jeweils ca. 1,2 kg in einem 1,5l Kneter der Firma Gumix S.A. hergestellt. Die Drehzahl betrug 30 U/min, der Füllgrad des Kneters betrug 70% und die Mischungstemperatur ca. 100°C. Die Grundmischung 1 enthielt 100 phr Naturkautschuk SMR10 und 1 phr Stearinsäure. Diese Grundmischung 1 wurde für ca. 5 min. im Kneter gemischt und aus dieser Mischung innerhalb einer Minute auf einem Doppelwalzwerk der Firma Rubicon Gummitechnik und Maschinentechnik GmbH ein Fell geformt. Die Grundmischung 2 enthielt zusätzlich 50 phr Ruß N110 und wurde entsprechend Grundmischung 1 gemischt. Grundmischung 3 entspricht Grundmischung 2 mit der Ausnahme, dass nur für 2 Min. im Kneter gemischt wurde. Grundmischung 4 entspricht ebenfalls Grundmischung 2 mit der Ausnahme, dass die Mischung nur für 1 Minute gemischt wurde. Durch die unterschiedlichen Mischweisen wurden unterschiedliche Verteilungs- und Deagglomerationszustände des Füllstoffs erhalten. Der Ruß N110 bestand in seiner Lieferform aus Pellets (agglomerierte Füllstoffeinheiten) die einen Durchmesser im Bereich von ≤ 2 mm aufwiesen. Die vier unterschiedlichen Mischungen wurden entsprechend Beispiel 1 nacheinander dem Extruder zugeführt und der relative Dämpfungskoeffizient $\alpha_{rel}.(t)$ sowie die Schallgeschwindigkeit v bestimmt. Bei der Berechnung von Intensität I(t) wurde hier auf die mittlere Intensität $I_{mittel}$ der Grundmischung 1 ohne Ruß zwischen t = 0 min. und t = 7 min. normiert. Fig. 6 zeigt die Messungen aller 4 Grundmischungen, wobei diese Mischungen nacheinander dem Extruder zugeführt und nacheinander gemessen wurden, siehe nachstehende Tabelle.

| Grundmischung | Prozesszeit [min] |
|---|---|
| 1 | 0-7 |
| 2 | 8-15 |
| 3 | 16-24 |
| 4 | 24-30 |

Aus Fig. 5 ist ersichtlich, dass die Grundmischung 2 eine deutlich größere Dämpfung des Ultraschalls aufweist als die Grundmischung 1 ohne Ruß. Die Grundmischung 3 mit einer Mischzeit von nur zwei Minuten und einem größeren Anteil an partikulären Verunreinigungen hat gegenüber Grundmischung 2 eine höhere Dämpfung. Zudem ist die Standardabweichung vom Mittelwert des relative Dämpfungskoeffizient der Grundmischung 3 deutlich größer als bei Grundmischung 2. Grundmischung 4 verfügt mit nur einer Minute Mischzeit über eine sehr geringe Deagglomeration des Füllstoffs, dass heißt sehr viele grobpartikuläre Verunreinigungen. Hier ist der realative Dämpfungskoeffizient sowie die Standardabweichung noch mal deutlich größer als bei Grundmischung 2 und 3. Aus Tabelle 2 und Fig. 5 ist eindrucksvoll ersichtlich, dass verschiedene Mischungen (Grundmischungen 1 bis 4) mit dem erfindungsgemäßen Verfahren eindeutig voneinander unterscheidbar sind. Mit diesem Verfahren kann daher der unterschiedlichen Verteilungsgrad und Deagglomerationszustand eindeutig nachgewiesen werden.

In Fig. 6 ist die Schallgeschwindigkeit v(t) in Abhängigkeit der Prozesszeit t dargestellt. Die Geschwindigkeit ergibt sich aus dem Quotienten der Wegstrecke s und der Laufzeit $t_{US}$, die der Nadelimpuls am benötigt um die Strecke s zwischen Sender und Empfänger zu überwinden.

$$v = s/t_{US}$$

Im Gegensatz dazu unterscheidet sich die Schallgeschwindigkeit der Grundmischungen mit Ruß nur wenig in Abhängigkeit der Mischzeit im Kneter (Fig. 6), da in den Grundmischungen 2-4 die Konzentrationen an Ruß im Detektionsvolumen gleich sind. Dagegen ist die Schallgeschwindigkeit der Grundmischung 1 ohne Ruß deutlich geringer. Damit können andere qualitätsbestimmende Kriterien als der mittels der zeitabhängigen Funktion des relativen Dämpfungskoeffizienten gemessene unterschiedliche Verteilungsgrad ausgeschlossen werden.

Die Geschwindigkeit v(t) verändert sich im Gegensatz zum relativen Dämpfungskoeffizienten nicht. Dies zeigt, dass dies ausschließlich auf die großpartikulare Verunreinigungen zurückzuführen ist und nicht auf Grundmischung.

Das Ergebnis zeigt, dass grobe Verunreinigungen mit dem erfindungsgemäßen Verfahren in einer Kautschukmischung erkannt werden können. Die Methode ist schnell. Ein Mischungsansatz von ca. 1 kg wurde innerhalb weniger Minuten ohne großen manuellen Aufwand untersucht. Die Methode ist zudem repräsentativ, da der Durchmesser des Schwingers von 7 mm des Ultraschallsensors knapp die Hälfte der Breite des Fließkanals von 15 mm abdeckt.

Zudem wurde insgesamt mehr als 4 kg von den verschiedenen Mischungen innerhalb von 30 Minuten untersucht.

Vergleichsversuche:

Die nach dem Stand der Technik bekannten Verfahren sehen 5 Messungen der Mooney-Viskosität für eine Mischung vor. Hier beträgt der Zeitaufwand ca. 30 min inklusive Probenvorbereitung. Für vier Mischungen beträgt der Zeitaufwand dann ca. zwei Stunden. Zudem kann die Ursache nicht direkt auf grobpartikuläre Verunreinigungen zurückgeführt werden. Hierzu müssen lichtmikroskopische Aufnahme gemacht werden. Lichtmikroskopische Aufnahmen benötigen ebenfalls einen Zeitaufwand von ca. 2 Minuten pro Stichprobe. Hier müssen zudem viele Stichproben genommen werden. Zudem ist es schwierig, lichtmikroskopische Aufnahmen von unvernetzten Kautschukmischungen herzustellen. Bei den beiden nach dem Stand der Technik bekannten Verfahren wird zudem deutlich weniger Material als mit dem Ultraschallverfahren untersucht. Hier wird ca. die Hälfte des Mischungsmaterials überprüft. Dies zeigt, dass das erfindungsgemäße Verfahren gegenüber den herkömmlichen Verfahren repräsentativ und weniger zeitaufwendig ist.

**Patentansprüche**

1.  Verfahren zur Bestimmung der Qualität einer unvernetzten Kautschukmischung durch Messung der Transmission des Ultraschalls über die Prozesszeit, **dadurch gekennzeichnet, dass** die Messung bei Temperaturen > 25°C und Frequenzen zwischen 0,5 MHz und 20 MHz durchgeführt wird.

**2.** Verfahren nach Anspruch 1, **dadurch gekennzeichnet, dass** zusätzlich die Schallgeschwindigkeit der unvernetzten Kautschukmischung über die Prozesszeit ermittelt wird.

**3.** Verfahren nach einem oder mehreren der Ansprüche 1 bis 2, **dadurch gekennzeichnet, dass** die unvernetzte Kautschukmischung ein Detektionsband aus mindestens einem Ultraschallsensorpaar durchläuft .

**4.** Verfahren nach einem oder mehreren der Ansprüche 1 bis 3, **dadurch gekennzeichnet, dass** das Detektionsband aus mindestens 2 nebeneinander angeordneten Ultraschallsensoren besteht, die über die gesamte Breite der unvernetzten Kautschukmischung angeordnet sind.

**5.** Vorrichtung enthaltend eine Anordnung aus einem Extruder, mindestens einem Detektionsband aus mindestens 2 nebeneinander angeordneten Ultraschallsensorpaaren und mindestens eine Auswerteeinheit zur Durchführung eines Verfahrens nach einem oder mehreren der Ansprüche 1 bis 4.

**6.** Verwendung der Vorrichtung nach Anspruch 5 zur Bestimmung der Qualität in einer unvernetzten Kautschukmischung.

**7.** Verwendung der Vorrichtung nach Anspruch 5 zur Herstellung einer unvernetzten Kautschukmischung.

**8.** Verwendung der nach einem oder mehreren der Ansprüche 1 bis 4 geprüften unvernetzten Kautschukmischung zur Herstellung von Reifen, Profilen, Dichtungen, Schläuchen, Bauteilen zur Vibrationskontrolle.

Fig. 1

(4)

(5)

Fig.2

(4) (4) (4) (4) (4)

(5)

(4) (4) (4) (4) (4)

Fig.3

(4)

(5)

(1)

(2)

(3)

Fig.4

Fig.5

Fig.6

EP 2 551 087 A1

Europäisches
Patentamt

European
Patent Office

Office européen
des brevets

**EUROPÄISCHER RECHERCHENBERICHT**

Nummer der Anmeldung

EP 11 17 5837

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X,D | EP 2 314 442 A1 (RHEIN CHEMIE RHEINAU GMBH [DE]) 27. April 2011 (2011-04-27) * Absatz [0059] - Absatz [0078] * ----- | 1-7 | INV. B29C47/92 G01N29/07 G01N29/11 G01N33/44 |
| X | DE 101 38 790 A1 (STARK WOLFGANG [DE]; DOERING JOACHIM [DE]; KELM JUERGEN [DE]) 20. Februar 2003 (2003-02-20) | 1,2 | |
| A | * Absatz [0015] - Absatz [0023]; Abbildungen 1-4,7 * ----- | 3-6 | |
| X | EP 2 179 835 A1 (SIKORA AG [DE]) 28. April 2010 (2010-04-28) * Absätze [0019], [0023]; Abbildungen 1,2 * ----- | 1-6 | |
| X | JAUNICH M ET AL: "Monitoring the vulcanization of elastomers: Comparison of curemeter and ultrasonic online control", POLYMER TESTING, ELSEVIER, Bd. 28, Nr. 1, 1. Februar 2009 (2009-02-01), Seiten 84-88, XP025768223, ISSN: 0142-9418, DOI: 10.1016/J.POLYMERTESTING.2008.11.005 [gefunden am 2008-11-21] | 1,2 | RECHERCHIERTE SACHGEBIETE (IPC) B29C G01N |
| A | * das ganze Dokument * ----- | 3-6 | |
| A | WO 2010/005375 A1 (REOSENSE AB [SE]; CRONVALL LEIF [SE]; LUNDBERG JONAS [SE]) 14. Januar 2010 (2010-01-14) * Seite 2, Zeile 10 - Seite 4, Zeile 11; Abbildungen 1,2 * ----- | 1-6 | |

Der vorliegende Recherchenbericht wurde für alle Patentansprüche erstellt

| Recherchenort | Abschlußdatum der Recherche | Prüfer |
|---|---|---|
| Den Haag | 29. November 2011 | Gilow, Christoph |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**                    EP 11 17 5837

In diesem Anhang sind die Mitglieder der Patentfamilien der im obengenannten europäischen Recherchenbericht angeführten
Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

29-11-2011

| Im Recherchenbericht angeführtes Patentdokument | Datum der Veröffentlichung | Mitglied(er) der Patentfamilie | Datum der Veröffentlichung |
|---|---|---|---|
| EP 2314442 A1 | 27-04-2011 | EP 2314442 A1<br>WO 2011048129 A1 | 27-04-2011<br>28-04-2011 |
| DE 10138790 A1 | 20-02-2003 | KEINE | |
| EP 2179835 A1 | 28-04-2010 | CN 101722650 A<br>DE 102009004946 A1<br>EP 2179835 A1<br>RU 2009138294 A<br>US 2010096767 A1 | 09-06-2010<br>29-04-2010<br>28-04-2010<br>27-04-2011<br>22-04-2010 |
| WO 2010005375 A1 | 14-01-2010 | CN 102084244 A<br>EP 2310845 A1<br>KR 20110027837 A<br>US 2011183422 A1<br>WO 2010005375 A1 | 01-06-2011<br>20-04-2011<br>16-03-2011<br>28-07-2011<br>14-01-2010 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5121629 A
- EP 2314442 A
- EP 2009058041 W
- DE 102008040138 A

**In der Beschreibung aufgeführte Nicht-Patentliteratur**

- **A. SCHRÖDER et al.** *Kautschuk, Gummi Kunststoffe,* 2008, vol. 11, 584-596
- **PAYNE, A.R. ; WATSON, W.F.** *Rubber Chem. and Technol.,* 1963, vol. 36 (1), 147-155
- **J. SCHNETGER.** Lexikon Kautschuktechnik. 2004, 388-391
- **J. SCHNETGER.** Lexikon Kautschuktechnik. 2004, 448-449
- **J. SCHNETGER.** Lexikon Kautschuktechnik. 2004, 557-558
- **J. KIRCHHOFF et al.** *Gummi Kunststoffe,* 2002, vol. 55, 373-381